## Europäisches Patentamt

## European Patent Office

## Office européen des brevets

(19)

(11) Veröffentlichungsnummer : **0 070 992**
**B1**

(12)

# EUROPÄISCHE PATENTSCHRIFT

(45) Veröffentlichungstag der Patentschrift :
09.01.85

(51) Int. Cl.⁴ : **C 12 Q    1/28, C 12 N    9/96**

(21) Anmeldenummer : **82104551.5**

(22) Anmeldetag : **25.05.82**

(54) Verfahren zur Stabilisierung von Peroxidase in einem flüssigen Medium und so stabilisiertes Medium.

(30) Priorität : **30.07.81 CH 4944/81**

(43) Veröffentlichungstag der Anmeldung :
**09.02.83 Patentblatt 83/06**

(45) Bekanntmachung des Hinweises auf die Patenterteilung : **09.01.85 Patentblatt 85/02**

(84) Benannte Vertragsstaaten :
**AT BE CH DE FR GB IT LI NL SE**

(56) Entgegenhaltungen :
EP-A- 0 024 578
US-A- 4 252 896
CHEMICAL ABSTRACTS, Band 92, Nr. 1, 7. Januar 19
80, Seite 276, Nr. 2753w, Columbus, Ohio, USA S.
INAGAKI: "Comparative study on 8 chromogens in
glucose determination by glucose oxidase-peroxidase system. II."
CHEMICAL ABSTRACTS, Band 92, Nr. 1, 7. Januar
1980, Seite 276, Nr. 2754x, Columbus, Ohio, USA S.
INAGAKI: "Comparative study on 8 chromogens in
glucose determination by glucose oxidase-peroxidase system. IV"
CHEMICAL ABSTRACTS, Band 92, Nr. 1, 7. Januar
1980, Seite 276, Nr. 2755y, Columbus, Ohio, USA S.
INAGAKI: "Comparative study on 8 chromogens in
glucose determination by glucose oxidase-peroxidase system. III."
CHEMICAL ABSTRACTS, Band 90, Nr. 13, 26. März
1979, Seite 228, Nr. 99575j, Columbus, Ohio, USA S.
INAGAKI et al.: "Determination of glucose by the
glucose oxidase-peroxidase method using 4-ami-
noantipyrine-Diazo Red RC chromogen"

(73) Patentinhaber : **F. HOFFMANN-LA ROCHE & CO.
Aktiengesellschaft**

**CH-4002 Basel (CH)**

(72) Erfinder : **Gallati, Harald, Dr.
Ingelsteinweg 13
CH-4143 Dornach (CH)**
Erfinder : **Brodbeck, Hans
Lindenstrasse 12
CH-4142 Münchenstein (CH)**

(74) Vertreter : **Kloter, Rudolf et al
Postfach 3255 Grenzacherstrasse 124
CH-4002 Basel (CH)**

**Beschreibung**

Das Enzym Peroxidase, insbesondere die Meerrettich-Peroxidase, ist sehr vielseitig anwendbar und kommt insbesondere zum Markieren von immunologischen Reaktionspartnern, z. B. Haptenen, Antigenen oder Antikörpern, für immunologische Testverfahren in Betracht. Da die Aktivität bzw. das Vorhandensein von Peroxidase leicht und einfach nachweisbar ist, wird Peroxidase bei immunologischen Verfahren besonders bevorzugt verwendet. Aus diesem Grunde enthalten denn auch kommerziell erhältliche Kits für ein Enzymimmunverfahren als wesentlichen Bestandteil ein immunologisch aktives Material, an das Peroxidase gekoppelt ist. Da derartige Kits vor der Verwendung unterschiedlich lange transportiert und gelagert werden, ist es notwendig, dass die Aktivität des Enzyms so lange wie möglich erhalten bleibt. Nun ist aber bekannt, dass das Enzym Peroxidase unabhängig davon, ob es an eine andere Komponente gebunden ist, speziell in niedrigen Konzentrationen nicht sehr beständig ist. Daher ist auch die Lagerstabilität gering, was die kommerzielle Bedeutung solcher Kits beeinträchtigt.

Gegenwärtig werden Peroxidase-Konjugate in einem Serum oder Serumprotein enthaltenden Reaktionsmedium aufbewahrt, welches z. B. ungefähr 20 % Ziegenserum enthalten kann. Hierbei hat sich gezeigt, dass die Anwesenheit des Serums die Stabilität des Konjugates erhöht. Andererseits wurde bei höheren Lagertemperaturen (z. B. 37 °C) und/oder nach längerer Lagerdauer (z. B. 6 Monate) auch beobachtet, dass das Serum zur Inaktivation der Peroxidase beiträgt, indem es Häminteile aus dem Enzym abspaltet. Diese Inaktivierung der Peroxidase ist offenbar durch die Häminteinteraktionen zwischen der Peroxidase und den Hämin-bindenden Proteinen des Serums bedingt.

In der US-A-4 252 896 wird vorgeschlagen, die Peroxidase durch Zusatz von 8-Anilino-1-naphthalinsulfonsäure zu stabilisieren.

Im Rahmen der vorliegenden Erfindung wurde nun gefunden, dass die Inaktivierung der Peroxidase durch die Zugabe von 4-Amino-antipyrin zum Medium wesentlich vermindert werden kann. Durch die Zugabe von 4-Amino-antipyrin zum Reaktionsmedium wird demgemäss die Stabilität der Peroxidase wesentlich verbessert.

Die vorliegende Erfindung betrifft demnach ein Verfahren zur Stabilisierung von Peroxidase, an welche ein immunologisch aktives Material gekoppelt ist, in einem Serum oder Serumprotein enthaltenden Medium, welches dadurch gekennzeichnet ist, dass man dem Medium 4-Amino-antipyrin zufügt.

Als Peroxidase kommt beim erfindungsgemässen Verfahren insbesondere die Meerrettich-Peroxidase in Betracht. Als Serum hat sich insbesondere Ziegenserum als bevorzugt erwiesen, doch kann z. B. auch foetales Kälberserum verwendet werden. Als Serumprotein kommt z. B. Albumin in Betracht.

Die Konzentration an 4-Amino-antipyrin im Medium beträgt vorzugsweise zwischen 10 mg bis 1 000 mg pro Liter, insbesondere 40 bis 300 mg pro Liter. Nach einem ganz besonders bevorzugten Aspekt wird das 4-Amino-antipyrin in einer Konzentration von 100 mg oder 200 mg pro Liter verwendet.

Das nachfolgende Beispiel erläutert die Erfindung.

Beispiel

0,1 mg/l (Ziegen) Anti-CEA-Peroxidase-Konjugat werden in 0,2 mol/l Natriumphosphatpuffer vom pH 6,5 mit 2 g/l Rinderserumalbumin, 20 % normalem Ziegenserum (inaktiviert bei 56 °C/30 Min.) sowie 0,5 g/l « Thimerosal » (Fluka) gelöst. Diese Anti-CEA-Peroxidaselösung wird in zwei Teile aufgeteilt. Einem Teil dieser Anti-CEA-Peroxidaselösung werden 0,2 g/l 4-Amino-antipyrin zugesetzt. Die beiden Anti-CEA-Peroxidaselösungen werden sterilfiltriert (Filter: 0,2 µ) und in Fraktionen von 20 ml in sterile Glasflaschen mit Schraubverschluss abgefüllt. Diese Anti-CEA-Peroxidaselösungen werden bei 2-8 °C resp. bei 37 °C aufbewahrt.

Nach bestimmten Zeitintervallen wird die Peroxidaseaktivität in den einzelnen Anti-CEA-Peroxidaselösungen bestimmt, indem zu 0,5 ml Substratpufferlösung (0,1 mol/l Natriumcitrat vom pH 5,0 mit 6 mmol/l $H_2O_2$ und 40 mmol/l o-Phenylendiamin) 0,050 ml der betreffenden Anti-CEA-Peroxidaselösung (vorverdünnt 1 : 20 in 9 g/l NaCl) zugemischt werden und indem man dann während 15 Minuten bei Raumtemperatur inkubiert. Durch Zugabe von 2,0 ml einer 1 N HCl wird die peroxidatische Reaktion abgestoppt und die Absorptionsdifferenz ($\Delta A_{492\ nm/RT/15\ Min.}$) photometrisch gemessen. Zur Bestimmung der stabilisierenden Wirkung des 4-Amino-antipyrins wird die prozentuale Restaktivität der bei 37 °C gelagerten Anti-CEA-Peroxidaselösung gegenüber jener, die bei 2-8 °C aufbewahrt wurde, berechnet.

Die folgende Tabelle fasst die Resultate des oben beschriebenen Versuchs zusammen und zeigt die stabilisierende Wirkung von 4-Amino-antipyrin.

| Prozentuale Restaktivität der Peroxidase nach : | | | | | |
|---|---|---|---|---|---|
| | 0 Tag | 7 Tage | 14 Tage | 21 Tage | 28 Tage |
| ohne 4-Amino-antipyrin | 100 % | 2 % | 0 % | 0 % | 0 % |
| mit 4-Amino-antipyrin | 100 % | 100 % | 77 % | 62 % | 58 % |

**0 070 992**

### Ansprüche

1. Verfahren zur Stabilisierung von Peroxidase, an welche ein immunologisch aktives Material gekoppelt ist, in einem Serum oder Serumprotein enthaltenden Medium, dadurch gekennzeichnet, dass man dem Medium 4-Amino-antipyrin zugibt.

2. Verfahren nach Anspruch 1, dadurch gekennzeichnet, dass die Peroxidase Meerrettich-Peroxidase ist.

3. Verfahren nach Anspruch 1 oder 2, dadurch gekennzeichnet, dass das Serum Ziegenserum ist.

4. Verfahren nach einem der Ansprüche 1 bis 3, dadurch gekennzeichnet, dass man 4-Amino-antipyrin in einer Konzentration von 10 mg bis 1 000 mg pro Liter zugibt.

5. Verfahren nach Anspruch 4, dadurch gekennzeichnet, dass man 4-Amino-antipyrin in einer Konzentration von 40 mg bis 300 mg pro Liter zugibt.

6. Verfahren nach Anspruch 5, dadurch gekennzeichnet, dass man 4-Amino-antipyrin in einer Konzentration von 100 mg oder 200 mg pro Liter zugibt.

7. Stabilisierte Peroxidase, an welche ein immunologisch aktives Material gekoppelt ist, in einem Serum oder Serumprotein enthaltenden Medium, dadurch gekennzeichnet, dass das Medium 4-Amino-antipyrin enthält.

### Claims

1. A method for the stabilization of peroxidase, to which is coupled an immunologically active material, in a medium containing serum or serum protein, characterized by adding 4-amino-antipyrine to the medium.

2. A method according to claim 1, characterized in that the peroxidase is horseradish peroxidase.

3. A method according to claim 1 or 2, characterized in that the serum is goat serum.

4. A method according to any one of claims 1 to 3, characterized in that 4-amino-antipyrine is added in a concentration of 10 mg to 1,000 mg per litre.

5. A method according to claim 4, characterized in that 4-amino-antipyrine is added in a concentration of 40 mg to 300 mg per litre.

6. A method according to claim 5, characterized in that 4-amino-antipyrine is added in a concentration of 100 mg or 200 mg per litre.

7. Stabilized peroxidase, to which is coupled an immunologically active material, in a medium containing serum or serum protein, characterized in that the medium contains 4-amino-antipyrine.

### Revendications

1. Procédé de stabilisation de peroxydase à laquelle est couplée une matière immunologiquement active, dans un milieu contenant du sérum ou une protéine sérique, caractérisé en ce qu'on ajoute au milieu de la 4-amino-antipyrine.

2. Procédé selon la revendication 1, caractérisé en ce que la peroxydase est la peroxydase de raifort.

3. Procédé selon les revendications 1 et 2, caractérisé en ce que le sérum est du sérum de chèvre.

4. Procédé selon l'une des revendications 1 à 3, caractérisé en ce qu'on ajoute la 4-amino-antipyrine à une concentration de 10 mg à 1 000 mg par litre.

5. Procédé selon la revendication 4, caractérisé en ce qu'on ajoute la 4-amino-antipyrine à une concentration de 40 mg à 300 mg par litre.

6. Procédé selon la revendication 5, caractérisé en ce qu'on ajoute la 4-amino-antipyrine à une concentration de 100 mg ou 200 mg par litre.

7. Peroxydase stabilisée à laquelle est couplée une matière immunologiquement active, dans un milieu contenant du sérum ou une protéine sérique, caractérisée en ce que le milieu contient de la 4-amino-antipyrine.